(19) **Europäisches Patentamt / European Patent Office / Office européen des brevets**

(11) **EP 1 736 203 B1**

(12) **FASCICULE DE BREVET EUROPEEN**

(45) Date de publication et mention de la délivrance du brevet:
**25.02.2009 Bulletin 2009/09**

(51) Int Cl.:
***A61N 1/362*** *(2006.01)*

(21) Numéro de dépôt: **06291003.9**

(22) Date de dépôt: **21.06.2006**

(54) **Dispositif médical implantable actif à stimulation biventriculaire et optimisation automatique de la configuration de stimulation**

Implantierbares medizinisches Gerät zur biventrikulären Stimulation und zur automatischen Optimierung der Anregungskonfiguration

Implantable medical device for biventricular stimulation and automatic optimisation of the pacing configuration

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **22.06.2005 FR 0506317**

(43) Date de publication de la demande:
**27.12.2006 Bulletin 2006/52**

(73) Titulaire: **ELA MEDICAL**
**92541 Montrouge (FR)**

(72) Inventeurs:
• **Plicchi, Gianni**
**40138 Bologna (IT)**

• **Marcelli, Emanuela**
**62100 Macerata (IT)**
• **Renesto, Fabrizio**
**10013 Borgofranco d'Ivrea (TO) (IT)**

(74) Mandataire: **Dupuis-Latour, Dominique et al**
**Bardehle Pagenberg Dost Altenburg Geissler**
**10, boulevard Haussmann**
**75009 Paris (FR)**

(56) Documents cités:
**WO-A-20/05011803        US-A1- 2003 208 240**
**US-A1- 2004 054 381**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

L'invention concerne les « dispositifs médicaux implantables actifs » tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

L'invention concerne plus particulièrement les dispositifs aptes à assurer une stimulation conjointe et permanente des ventricules gauche et droit afin de resynchroniser ces derniers, technique dite "CRT" (*Cardiac Re-synchronization Therapy)* ou "BVP" *(Bi-Ventricular Pacing).*

En effet, en alternative ou en complément au traitement des troubles du rythme cardiaque, il a été proposé de traiter par stimulation biventriculaire certains troubles de contraction myocardique observés chez des patients en insuffisance cardiaque, ces troubles pouvant être spontanés ou induits par une stimulation traditionnelle. On pourra notamment se référer à l'étude de J. C. Daubert et coll., Stimucoeur, 25, n°3, pp. 170-176 qui fait le point des travaux sur ce sujet. Cette thérapie a permis d'observer des résultats souvent spectaculaires pour des patients en insuffisance cardiaque en classe III non améliorée par les traitements classiques.

Un stimulateur CRT est par exemple décrit dans le EP-A-1 108 446 (ELA Medical), qui décrit un dispositif permettant d'appliquer entre les deux stimulations ventriculaires un délai interventriculaire variable, ajusté de manière à resynchroniser la contraction des ventricules avec optimisation fine de l'état hémodynamique du patient.

Les dispositifs CRT utilisés actuellement sont le plus souvent des prothèses dites "multisite" dans lesquelles des électrodes sont placées en une pluralité de sites respectifs distincts comportant au moins un site auriculaire en plus des deux sites ventriculaires droit et gauche, comme dans le cas des prothèses "triple chambre" (double stimulation ventriculaire et détection/stimulation auriculaire droite) ou "quadruple chambre" (double stimulation ventriculaire et double détection/stimulation auriculaire).

On appellera "sites de stimulation" l'emplacement physique des électrodes intracardiaques par rapport au tissu du myocarde ; généralement, ces sites ne peuvent être choisis qu'au moment de l'implantation, par un positionnement approprié des électrodes. Il est important de vérifier l'efficacité des sites choisis, en raison de l'influence possible à long terme de l'efficacité de la thérapie de resynchronisation. Dans certains cas, le dispositif multisite comporte plusieurs électrodes placées dans une même cavité, et une modification du site de stimulation dans cette cavité est alors possible par une commutation interne du dispositif.

La notion de "séquence de stimulation" se référera, d'une part, à l'ordre suivant lequel les impulsions de stimulation sont appliquées au coeur (par exemple d'abord oreillette, puis ventricule gauche, puis ventricule droit) et, d'autre part, aux intervalles de temps séparant l'application de ces impulsions successives. La séquence de stimulation est paramétrée à l'implantation, et peut être si nécessaire modifiée par la suite par des commutations internes appropriées du dispositif et un ajustement des paramètres de séquencement des impulsions.

On appellera "configuration de stimulation" la combinaison des caractéristiques relatives aux "sites de stimulation" et de celles relatives à la "séquence de stimulation".

Le problème de l'invention est celui de l'optimisation de la stimulation biventriculaire par sélection des sites de stimulation les plus appropriés et de la meilleure séquence de stimulation de ces sites.

Cette optimisation est bien sûr effectuée au moment de l'implantation, mais il serait souhaitable de la réitérer par la suite à intervalles périodiques, pour suivre l'évolution de l'état hémodynamique général du patient, en particulier lors d'une amélioration ou, au contraire, d'une aggravation de l'état du myocarde.

Il existe divers moyens pour évaluer le degré d'efficacité d'une stimulation biventriculaire. La plupart du temps, il s'agit de techniques échographiques, qui doivent toujours être mises en oeuvre en milieu hospitalier par un personnel qualifié. Elles sont de ce fait coûteuses et ne peuvent être appliquées aussi souvent que cela serait utile ou nécessaire sans interférer avec la vie quotidienne du patient.

Le EP-A-1 108 446 précité propose une solution consistant à évaluer le degré de synchronisation des contractions des ventricules droit et gauche par une mesure de bioimpédance intracardiaque, donnée représentative du débit cardiaque et donc de la fraction d'éjection, considérée comme étant le paramètre hémodynamique de référence.

Le WO-A- 2005/011803 décrit un dispositif de stimulation biventriculaire dont l'efficacité est évaluée en analysant les mouvements de la cloison séparant les deux ventricules. Un capteur d'accélération est fixé perpendiculairement à cette cloison, et une boucle de rétraction modifie le fonctionnement du stimulateur de manière à minimiser le signal capté , qui reflète le degré de désynchronisation des ventricules.

La présente invention propose une autre approche de l'optimisation de la stimulation biventriculaire, mettant en oeuvre une analyse de l'accélération endocardiaque, plus précisément une analyse des pics d'accélération endocardiaque.

En effet, les études cliniques qui ont été menées indiquent que l'accélération endocardiaque est un paramètre permettant de fournir des informations très complètes sur l'état fonctionnel du myocarde, aussi bien dans le cas d'un fonctionnement normal que d'un fonctionnement déficient : l'accélération endocardiaque, qui est mesurée par un accéléromètre directement en contact avec le muscle cardiaque (généralement, mais non exclusivement, à l'apex ventriculaire droit), reflète en effet très précisément et en temps réel les phénomènes concourant au fonctionnement mécanique du coeur.

Plus précisément, le EP-A-0 515 319 (Sorin Biomedica Cardio SpA) enseigne la manière de recueillir un signal d'accélération endocardiaque au moyen d'une sonde endocavitaire pourvue d'une électrode distale de stimulation implantée au fond du ventricule et intégrant un micro-accéléromètre permettant de mesurer l'accélération endocardiaque.

Le signal d'accélération endocardiaque ainsi recueilli au cours d'un cycle cardiaque forme notamment deux pics, correspondant aux deux bruits majeurs qu'il est possible de reconnaître dans chaque cycle d'un coeur sain :

- le premier pic d'accélération endocardiaque ("PEA I") correspond à la fermeture des valvules mitrale et tricuspide, au début de la phase de contraction ventriculaire isovolumique (systole). Les variations de ce premier pic sont étroitement liés aux variations de la pression dans le ventricule (l'amplitude du pic PEA I étant, plus précisément, corrélée au maximum positif de la variation de pression dP/dt dans le ventricule gauche) et peuvent donc constituer un paramètre représentatif de la contractilité du myocarde, elle-même liée au niveau d'activité du système sympathique.
- le second pic d'accélération endocardiaque ("PEA II"), quant à lui, correspond à la fermeture des valvules aortique et pulmonaire, au moment de la phase de relaxation ventriculaire isovolumique. Ce second pic, qui est produit par la décélération brusque de la masse sanguine en mouvement dans l'aorte, constitue un paramètre représentatif de la pression sanguine périphérique au début de la diastole.

Le EP-A-0 655 260 (Sorin Biomedica Cardio SpA) décrit une manière de traiter le signal d'accélération endocavitaire délivré par le capteur situé en bout de sonde pour en dériver deux valeurs respectives liées à ces pics d'accélération endocardiaque, utiles notamment pour la détection de troubles cardiaques et le déclenchement ou non d'une thérapie de défibrillation.

Le dispositif de l'invention est du type général décrit par le WO-A-2005/011803 précité correspondant au préambule de la revendication 1, et comprend les éléments énoncés dans la partie caractéristique de cette revendication 1. Les sous-revendications visent des modes de réalisation avantageux.

On va maintenant décrire un exemple de mise en oeuvre de l'invention, en référence aux dessins annexés.

La figure 1 est un chronogramme montrant, au cours de trois cycles cardiaques successifs, les variations de l'accélération endocavitaire ainsi que de l'électrogramme et de l'électrocardiogramme de surface.

La figure 2 montre chez différents patients, sains ou malades, des caractéristiques de variation de l'amplitude du premier pic d'accélération endocardiaque en fonction du délai auriculo-ventriculaire.

La figure 3 illustre chez un même patient une forme normalisée de la caractéristique de la figure 2, pour deux configurations de stimulation différentes.

On va maintenant décrire un exemple de réalisation du dispositif de l'invention.

En ce qui concerne ses aspects logiciels, elle peut être mise en oeuvre par une programmation appropriée du logiciel de commande d'un stimulateur connu, par exemple de type stimulateur cardiaque triple ou quadruple chambre intégrant un mode de resynchronisation des ventricules. L'invention peut notamment être appliquée aux dispositifs implantables commercialisés par ELA Médical, Montrouge, France tels que les appareils *Symphony* et *Rhapsody.* Il s'agit de dispositifs à microprocesseur programmables comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

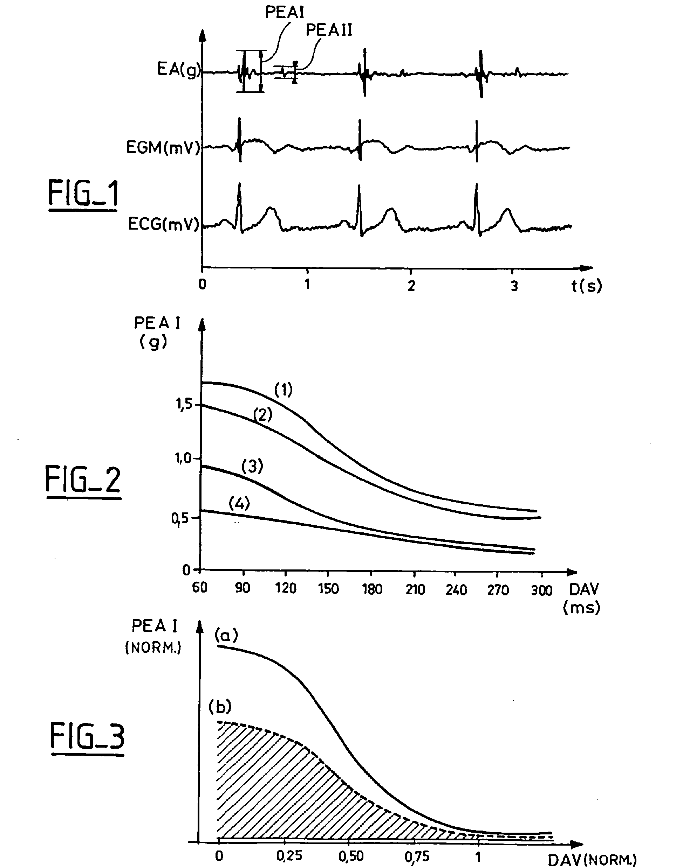

Sur la figure 1, on a représenté (courbe du haut), les variations de l'accélération endocardiaque (EA), mesurée par un capteur tel que celui décrit dans le EP-A-0 515 319 précité, intégré à une tête de sonde endocavitaire placée au fond du ventricule. On a également illustré sur cette figure les tracés de l'électrogramme (EGM), c'est-à-dire du signal électrique recueilli par l'électrode distale de ce même capteur, et d'un électrocardiogramme de surface (ECG) correspondant, au cours de trois cycles cardiaques consécutifs. Comme on l'a expliqué plus haut le tracé de l'accélération présente deux complexes successifs ou pics d'accélération endocardiaque (PEA), dont l'amplitude et la durée peuvent être déterminées par un traitement approprié du signal délivré par le capteur d'accélération, comme décrit dans le EP-A-0 655 260 précité. Par "amplitude du pic" on entendra la valeur maximale crête-à-crête du signal d'accélération séparant les deux extrema, positif et négatif, correspondant aux écarts PEA I et PEA II indiqués sur le chronogramme de la figure 1. Par "durée du pic" on entendra l'intervalle de temps séparant le début et la fin du complexe.

La présente invention propose d'utiliser des paramètres liés à l'accélération endocardiaque ainsi recueillie pour déterminer une configuration de stimulation optimale pour le patient, aussi bien au moment de l'implantation qu'ultérieurement. Divers paramètres peuvent être utilisés à cette fin, notamment : l'amplitude du PEA I et/ou du PEA II, la durée du PEA I et/ou du PEA II, l'intervalle de temps séparant le PEA I du PEA II consécutif associé, l'intervalle de temps séparant le PEA II du PEA I consécutif du cycle cardiaque suivant.

Dans un mode de mise en oeuvre simple de l'invention, le praticien utilise l'amplitude du PEA I pour le guider lors de l'implantation. La suite d'opération est alors la suivante :

a) implantation des sondes atriale et ventriculaire droite (la sonde ventriculaire droite comportant un capteur accélérométrique incorporé) ;

b) placement de la sonde ventriculaire gauche en un site approprié ;

c) raccordement du générateur et application d'une courte séquence d'impulsions de stimulation ;

d) enregistrement de l'amplitude du PEA I pendant la stimulation ;

e) répétition des étapes b) à d) en modifiant à chaque fois la position (site de stimulation) de la sonde ventriculaire gauche ;

f) choix de la position qui maximise l'amplitude du PEA I.

En variante, l'étape d) d'enregistrement de l'amplitude du PEA I peut être complétée ou remplacée par l'enregistrement d'un ou plusieurs autres paramètres, par exemple la mesure de l'amplitude et de la durée du PEA I, ou encore la mesure de l'amplitude et/ou de la durée du PEA II et de l'intervalle PEA I à PEA II (intervalle représentatif de la durée d'éjection).

Lorsque plusieurs paramètres sont combinés, un indice de performance composite est calculé à partir des différentes mesures, pour permettre d'évaluer la configuration testée, le choix final étant celui de la configuration qui maximise cet indice de performance.

Il est ainsi possible d'utiliser un indice de performance calculé par exemple (mais de façon aucunement limitative) de la manière suivante :

$$\text{Indice} = \text{amplitude}_{\text{PEA I}} * [\text{intervalle PEA I-PEA II}] / \text{durée}_{\text{PEA I}}$$

L'évaluation de l'indice de performance peut être opérée cycle à cycle, en temps réel pendant le déplacement de l'électrode ventriculaire gauche sur différents sites de stimulation. Le positionnement de la sonde ventriculaire droite et/ou la séquence de stimulation peuvent être également modifiés.

Le choix des sites de stimulation n'est en principe pas modifiable après l'implantation. Toutefois, certains dispositifs sont pourvus de sondes, notamment bipolaires, comportant plusieurs électrodes dans une même cavité. Il est alors possible de modifier si nécessaire le choix des sites de stimulation par des commutations internes du dispositif, ce dernier étant par exemple du type décrit dans le EP-A-0 925 806 (ELA Medical) qui divulgue un dispositif multisite pourvu de moyens de sélection de configurations d'électrodes, notamment pour améliorer des paramètres cardiaques tels que le débit. L'homme du métier pourra transposer sans difficulté les enseignements de ce document au cas d'un dispositif de stimulation biventriculaire.

Des modifications peuvent être apportées si nécessaire à la séquence de stimulation après implantation, de façon automatique ou lors d'un contrôle par un praticien dans le cadre du suivi du patient.

On va maintenant exposer, en référence aux figures 2 et 3, un mode de réalisation préférentiel de l'invention consistant à obtenir, pour chaque configuration de stimulation, une caractéristique PEA I / DAV en exécutant un balayage du DAV tout en enregistrant les amplitudes du PEA I.

La figure 2 montre diverses caractéristiques ainsi obtenues pour plusieurs patients. Chez des patients sains, correspondant aux courbes relevées en (1) et (2), l'amplitude du PEA I présente une allure sigmoïde caractéristique lorsque l'on fait varier le DAV entre deux extrêmes, typiquement entre 60 et 300 ms. On peut interpréter ces courbes en considérant que l'amplitude décroissante du PEA I pour des DAV croissants est déterminée par deux facteurs principaux, à savoir :

- la "réserve de contractilité" du myocarde, correspondant au niveau de la ligne de base (valeur limite de PEA I pour les DAV longs), et
- le "bruit" produit par les valves cardiaques, principalement la valve mitrale, qui détermine l'élévation du niveau d'amplitude au-dessus de cette ligne de base, pour les DAV les plus courts.

Pour que la seconde composante puisse être significativement présente, il est nécessaire que la première le soit déjà, car c'est la contractilité du myocarde qui est la "force d'entraînement" pour tous les phénomènes mécaniques se produisant pendant le cycle cardiaque.

En cas d'insuffisance cardiaque, lorsque la réserve de contractilité est marginale, la réduction du remplissage ventriculaire pour les DAV les plus courts provoque une chute de contractilité résultant de la loi de Frank-Starling. Pour des DAV courts on obtient alors, comme on peut le voir sur les courbes (3) et (4) de la figure 2, une augmentation de l'amplitude du PEA bien moindre que chez les patients sains, cette augmentation étant quelquefois même à peine perceptible.

Il résulte de ce qui précède que, dans le cas d'une stimulation biventriculaire, une configuration de stimulation efficace se traduit par une accentuation marquée de la caractéristique PEA I / DAV pour les DAV courts.

Un indice de performance approprié peut ainsi être établi à partir des valeurs prises par l'amplitude du PEA I pour différents DAV.

Selon l'invention, on choisit comme indice de performance l'aire située sous cette caractéristique, valeur qui sera une bonne indication de la proportion de fibres myocardiques contribuant à la systole, procurant donc une contractilité cardiaque accrue mesurée par l'amplitude du PEA I.

La figure 3 illustre en (a) et (b) deux caractéristiques PEA I / DAV obtenues chez un même patient pour deux configurations de stimulation différentes.

Pour faciliter les calculs, la variation du DAV est de préférence normalisée de 0 à 1, correspondant aux deux extrêmes de la variation, et un algorithme détermine l'aire située sous la caractéristique relevée durant le balayage du DAV (aire hachurée, sur la figure 3). La configuration optimale est définie comme celle procurant la valeur d'aire la plus grande : ainsi, sur la figure 3 la configuration correspondant à la caractéristique (a) est considérée comme procurant une efficacité supérieure à celle correspondant à la configuration de la caractéristique (b).

L'indice de performance constitué par cette valeur d'aire pourra être utilisé, comme expliqué plus haut, pour choisir la configuration de simulation la plus appropriée au moment de l'implantation et ultérieurement, soit automatiquement par le dispositif soit sous le contrôle d'un praticien lors d'un examen de suivi du patient.

Cet indice de performance peut être modifié pour inclure, outre le paramètre d'aire A dérivé de la caractéristique PEA I / DAV, d'autres paramètres d'accélération endocardiaque, par exemple la durée $d_{REMP}$ du remplissage ventriculaire, et/ou la durée $d_{PEA\,I}$ du complexe PEA I, pour donner par exemple (mais de façon aucunement limitative) un indice calculé de la manière suivante :

$$\text{Indice} = A * d_{REMP} / d_{PEA\,I}$$

Un tel indice composite prendra une valeur élevée avec les configurations de stimulation qui produisent une aire plus grande et une durée de remplissage plus longue, et qui en même temps minimisent la durée du complexe PEA I.

L'indice de performance peut également être déterminé à partir d'un valeur moyenne de l'amplitude du PEA I, ce qui en fait correspond à l'aire de la caractéristique PEA I / DAV avec une variation du DAV normalisée de 0 à 1.

D'autres paramètres d'accélération endocardiaque peuvent être également introduits dans cet indice, tels que l'amplitude du PEA II, ou l'écart entre les valeurs maximum et minimum prises par l'amplitude du PEA I lorsque le DAV varie sur l'intervalle choisi.

Des quantités autres que celles liées à l'accélération endocardiaque peuvent également être introduites dans l'indice de performance, par exemple la fréquence cardiaque au repos, ou encore le niveau d'activité physique du patient déterminé par un capteur d'activité ou un capteur de ventilation-minute.

## Revendications

**1.** Un dispositif médical implantable actif à stimulation biventriculaire, comportant :

- des moyens de recueil d'un signal d'accélération endocardiaque (EA) comprenant un capteur endocavitaire d'accélération, en contact avec le myocarde, et
- des moyens d'analyse de signal, aptes à déterminer au moins une valeur de pic fonction de l'un et/ou de l'autre de deux pics de l'accélération endocardiaque au cours d'un cycle donné, ces deux pics comprenant un premier pic (PEA I) lors de la phase de contraction ventriculaire isovolumique et un second pic (PEA II) lors de la phase de relaxation ventriculaire isovolumique, et
- des moyens d'aide à la recherche d'une configuration de stimulation optimale,

ladite recherche de configuration de stimulation comprenant la sélection des sites de stimulation et/ou la modification de la séquence d'application des impulsions de stimulation aux différents sites de stimulation et/ou la modification de l'(les) intervalle(s) de temps séparant l'application des impulsions de stimulation aux différents sites de stimulation,

lesdits moyens d'aide à la recherche d'une configuration de stimulation optimale comprenant des moyens évaluateurs, aptes à déterminer pour chaque configuration de stimulation un indice de performance respectif dérivé de la (des) valeur(s) de pic,

- ledit capteur endocavitaire d'accélération étant destiné à être placé en fond de ventricule ;
- les moyens évaluateurs comprenant des moyens de balayage, aptes à faire varier de manière contrôlée le délai atrio-ventriculaire (DAV) séparant un événement auriculaire, spontané ou stimulé, d'une stimulation ventriculaire consécutive ;
- lesdits moyens d'aide à la recherche d'une configuration de stimulation optimale étant des moyens pour sélectionner la configuration de stimulation maximisant ledit indice de performance, dispositif **caractérisé en ce que:**

- les moyens évaluateurs sont aptes à dériver l'indice de performance à partir de l'aire définie sous la caractéristique de la (des) valeur(s) de pic durant la variation de ce délai atrio-ventriculaire.

2. Le dispositif de la revendication 1, dans lequel ladite valeur de pic comprend l'amplitude du premier pic.

3. Le dispositif de la revendication 2, dans lequel ladite valeur de pic comprend l'amplitude du premier pic combinée à au moins l'une des valeurs du groupe comprenant : la durée du premier pic ; l'amplitude du second pic ; l'intervalle de temps séparant le premier pic du second pic consécutif ; et l'intervalle de temps séparant le second pic du premier pic consécutif.

**Claims**

1. An implantable active medical device for biventricular stimulation, comprising

- means for capturing an endocardiac acceleration signal (EA) comprising an endocavitary acceleration sensor in contact with the myocardium,
- means for signal analysis, able to determine at least one peak value as a function of either or both of two peaks of the endocardiac acceleration during a given cycle, these two peaks comprising a first peak (PEA I) during the isovolumic ventricular contraction phase and a second peak (PEA II) during the isovolumic ventricular relaxation phase, and
- aids for searching for a configuration of optimal stimulation, said searching for a stimulation configuration comprising selecting the stimulation sites and/or modifying the application sequence of the stimulation impulsions to the different stimulation sites and/or modifying the time interval/intervals separating the applications of the stimulation impulsions to the different stimulation sites,
- said aids for searching for a configuration of optimal stimulation comprising evaluation means able to determine for each stimulation configuration a respective performance index from the peak value(s),
- wherein said endocavitary acceleration sensor is to be arranged at the bottom of the ventricle,
- wherein the evaluation means comprise scanning means able to vary in a controlled manner the atrio-ventricular delay (DAV) separating an auricular, spontaneous or stimulated event, from a consecutive ventricular stimulation ;

**characterized in that**

- the valuating means are able to derive the performance index from the area defined under the characteristic curve of the peak value(s) during variation of this atrio-ventricular delay,
- wherein said aids for searching for a configuration of optimal stimulation are means for selecting the stimulation configuration that will maximize said performance index.

2. The device of Claim 1, wherein said peak value comprises the amplitude of the first peak.

3. The device of Claim 2, wherein said peak value comprises the amplitude of the first peak combined with at least one of the values from the group comprising the duration of the first peak ; the amplitude of the second peak ; the time interval separating the first peak from the second consecutive peak ; and the time interval separating the second peak from the first consecutive peak.

**Patentansprüche**

1. Implantierbare, aktive medizinische Einrichtung mit Doppelkammerstimulation, umfassend

   - Mittel zur Erfassung eines endokardialen Beschleunigungssignals (EA) umfassend einen endokavitären Beschleunigungssensor im Kontakt mit dem Myokard,
   - Mittel zur Signalanalyse, die zur Bestimmung wenigstens eines Spitzenwerts geeignet sind, die Funktion des einen und/oder anderen der zwei Spitzenwerte der endokardialen Beschleunigung während eines gegebenen Zyklus ist, worin jene zwei Spitzenwerte einen ersten Spitzenwert (PEA I) während der Phase der isovolumischen Kammerkontraktion und einen zweiten Spitzenwert (PEA II) während der Phase der isovolumischen Kammerrelaxation umfassen, und
   - Hilfsmittel, die geeignet sind, der Suche nach einer optimalen Stimulationsgestaltung zu helfen ; worin besagte Suche nach einer Stimulationsgestaltung den Auswahl der Stimulationsstellen und/oder die Änderung der Abfolge der an die verschiedenen Stimulationsorte angelegten Stimulationsimpulse und/oder die Änderung des/der Zeitintervalls/e zwischen der Anlegung der Stimulationsimpulse an die verschiedenen Stimulationsorte umfasst ; worin besagte Hilfsmittel, die geeignet sind, der Suche nach einer optimalen Stimulationsgestaltung zu helfen, Abschätzungsmittel umfassen, die geeignet sind, für jede Stimulationsgestaltung einen respektiven, von dem/der Spitzenwert/e ableitenden Leistungsindex zu bestimmen,
   - worin besagte endokavitäre Beschleunigungssensor zur Anordnung im Kammerboden bestimmt ist,
   - worin besagte Abschätzungsmittel Abtastmittel umfassen, die geeignet sind, die atrioventrikuläre Verzögerung (DAV) zwischen einem aurikulären, spontanen bzw. stimulierten Ereignis und einer konsekutiven Ventrikelstimulation kontrolliert zu verändern,

   **dadurch gekennzeichnet, dass**

   - die Abschätzungsmittel geeignet sind, den Leistungsindex von der unter der Kennlinie des/der Spitzenwerts/werte bestimmten Fläche während der Änderung dieser atrioventrikulären Verzögerung abzuleiten,
   - worin besagte Hilfsmittel, die geeignet sind, der Suche nach einer optimalen Stimulationsgestaltung zu helfen, Mittel zum Auswahl der den besagten Leistungsindex maximisierenden Stimulationsgestaltung sind.

2. Einrichtung nach Anspruch 1, worin besagter Spitzenwert die Amplitude der ersten Spitze umfasst.

3. Einrichtung nach Anspruch 2, worin besagter Spitzenwert die wenigsten mit einer der Werte der Gruppe umfassend die Dauer der ersten Spitze, die Amplitude der zweiten Spitze, das Zeitintervall zwischen der ersten Spitze und der konsekutiven zweiten Spitze und das Zeitintervall zwischen der zweiten Spitze und der ersten konsekutiven Spitze kombinierte Amplitude der ersten Spitze umfasst.

PEAI

PEAII

EA(g)

EGM(mV)

ECG(mV)

FIG_1

0        1        2        3      t(s)

PEA I
(g)

1,5

(1)

(2)

1,0

FIG_2

(3)

(4)

0,5

0

60   90   120  150  180  210  240  270  300   DAV
(ms)

PEA I
(NORM.)

(a)

(b)

FIG_3

0    0,25   0,50   0,75    1     DAV(NORM.)

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1108446 A
- WO 2005011803 A
- EP 0515319 A
- EP 0655260 A
- EP 0925806 A

**Littérature non-brevet citée dans la description**

- **J. C. DAUBERT.** *Stimucoeur,* vol. 25 (3), 170-176